# NEW EUROPEAN PATENT SPECIFICATION

(11) **EP 0 230 980 B2**
(45) Date of publication and mention of the opposition decision: **20.03.1996**
(45) Mention of the grant of the patent: 24.07.1991
(21) Application number: 87100811.6
(22) Date of filing: 21.01.1987
(51) Int. Cl.: A61K 38/19

(54) **Pharmaceutical agent for promoting the recovery of hemopoietic capacity**
Pharmazeutischer Stoff für die Förderung der Wiederherstellung der hämatopoietischen Fähigkeit
Composé pharmaceutique promouvant le rétablissement de la capacité hémopoiétique

(30) Priority: 22.01.1986 JP 10280/86
(43) Date of publication of application: 05.08.1987
(73) Proprietor: Chugai Seiyaku Kabushiki Kaisha, Tokyo (JP)
(72) Inventor: Tamura, Masahiko, Takatsu-ku Kawasaki-shi Kanagawa-ken (JP); Hattori, Kunihiro, Mitaka-shi Tokyo (JP)
(74) Representative: VOSSIUS & PARTNER

(56) References cited:
- EP-A- 0 169 566
- EP-A- 0 169 566
- EP-A- 0 215 126
- JP-A- 23 777
- JP-A- 153 273
- UT M D Anderson, Symposium on Fundamental Cancer Research, vol. 37 "Mediators in Cell Growth and Differentiation". Moore et al.
- Proc. Natl. Acad. Sci. USA, vol. 82 pp1526-1530. March 1985 Welte et al.
- Souza et al., Science 232, 61-65 . 1986.
- Abstracts of 28th Meeting of the Japanese Society of Clinical Hematology, October 1986.
- Clark et al., "The Human Hematopoietic Colony. Stimulating Factors, Science 236 (1987), pp 1229-1237
- Kodo et al., "Clinical Application of G-CSF", Zoketsu Inshi (Hematopoietic Factor) 1 (1990), pp 51-56 and partial english translation
- Tamura et al., "acceleration of the hematopoietic Reconstitution in Mice undergoing Bone Marrow Transplantation by Recombinant hG-CSF", Transplantation 1 (1991) pp 1166-1170

## Description

The present invention relates to the use of a human granulocyte colony stimulating factor (hereinafter abbreviated as human G-CSF) for the preparation of a pharmaceutical composition for the recovery of hemopoietic capacity after bone marrow transplantation by promoting the increase in CFU-S.

Bone marrow transplantation (hereinafter BMT) is a technique intended to treat congential or acquired hemopoietic disorders in a patient by transplanting his own bone marrow or that of another healthy person.

BMT has recently come to be chosen as a treatment for patients with certain types of cancer and hematological disorders such as leukemia and malignant lymphoma because of the improvement it can achieve in the survival of bone marrow transplant recipients [see Rinsho to Kenkyu, 6l, l480 - l487 (l984) and Experimental Hematology, l2, 205-2l5 (l984)].

In spite of its efficacy, BMT has several problems associated with its clinication application. A major concern is that it has a potential for causing complications, such as infections immediately after BMT, interstitial pneumonia (IP) and graft-versus-host disease (GVHD).

Infections will occur in the early granulocytopenic period after BMT and isolation in a laminar airflow room is currently adopted to prevent this problem. It takes at least three weeks and even one month or more for the hemopoietic capacity of the patient to return to the normal levels. Although isolation in a laminar airflow room is efficacious for the prevention of serious infections, it is also very expensive for the patients. In addition, treatment in this room is quite laborious for medical personnel.

Development of IP often occurs after marrow engraftment. In order to prevent IP, sulfamethoxazole-trimethoprim is presently administered but this drug causes marrow suppression and is suitable only for patients who have fully recovered their hematopoietic capacity [see Rinsho Meneki, l5, 9, 700 - 707 and 687 - 699 (l983): and Experimental Hematology, l2, 205 - 2l5 (l984)].

Acute GVHD, which occurs after successful taking of graft, is the type of GVHD that requires most care. Methotrexate that has been administered for preventive purposes causes marrow suppression as does sulfamethoxazoletrimethoprim. Cyclosporin A (CSA) which has recently been added to the regimen for the treatment of acute GVHD has a problem associated with strong renal toxicity [see Rinsho to Kenkyu, 6l, 5, l480 - l487 (l984)].

In any event, it is strongly desired for bone marrow transplant recipients to restore their hematopoietic capacities as early as possible. However, in the absence of any pharmaceutical drug that is capable of meeting this need, the only way available today is to wait for spontaneous recovery of the patient's hematopoietic capacity.

In order to find a way to get around this impasse, the present inventors made concerted efforts and reached the idea of utilizing the pure human G-CSF which they previously succeeded in preparing and for patent of which they filed many applications. In order to put this concept into practice, the present inventors further proceeded with their studies.

CSFs are a series of factors that act on the progenitor cells in human or animal bone marrow cells in such a manner that they induce the fissiparity and differentiation of such progenitor cells into monocyte-macrophage and/or granulocyte [see Metcalf et al., Experimental Hematology, I, l85 (l973)]. A lot of reports have also been written on the topic of human CSF [e.g. Stanley et al., Federal Proceedings, 35, 2272 (l975) and Burgess et al., Blood, 49, 573 (l977), to name just a few].

However, the CSF described in these reports is not completely pure and no method has been established that is capable of large-scale preparation of CSF in a pure and homogeneous state. In order to develop a pharmaceutical drug having the ability to ensure early hemopoietic recovery after BMT, large-scale preparation of a pure and homogeneous G-CSF is a prerequisite and all of the problems associated with this need have been solved by the present inventors who have filed many patent applications on this success which include for example, EP No. 86 11 3671.1 and EP-A-0 169 566 which discloses the isolation and characterisation of hG-CSF and its ability to promote the differentiation and proliferation of bone marrow cells to granulocytes. In EP-A-0 215 126 pharmaceutical compositions containing 0.1-500 µg of hG-CSF are described as infection protective agents. All of these documents are to be used in the assessment of novelty only.

The present inventors conducted an experiment wherein the pure human G-CSF described in these applications was administered to mice daily; the data obtained in this experiment showed that said G-CSF caused enhanced hemopoietic capacity (see Experiment l to be described later in this specification).

The present inventors carried out another experiment in order to check to see whether the G-CSF could be used as a pharmaceutical drug to promote the recovery of hemopoietic capacity following BMT. A significant increase in CFU-S was observed in the G-CSF treated group. This demonstrated the ability of G-CSF to promote the recovery of hemopoietic capacity after BMT (see Experiment 2).

The present inventors also conducted an experiment using a model of retarded recovery of hemopoietic capacity; in this experiment, a control group achieved a survival rate of 33% whereas a G-CSF treated group attained a much higher level of 75%. This result is induced by the ability of G-CSF to promote the recovery of hemopoietic capacity (see Experiment 3).

The present invention has been accomplished on the basis of these findings.

The present invention provides a pharmaceutical agent that contains a human G-CSF as the effective ingredient and which is capable of promoting the recovery of hemopoietic capacity after BMT by promoting the increase in CFU-S.

The human G-CSF used as the active ingredient of the pharmaceutical agent of the present invention may be derived from any origin that is capable of producing human G-CSF. It is preferable to use the following two types of human G-CSF that were obtained by the methods on which patent applications were previously applied by the present inventors:
(1) human G-CSF having the following physicochemical properties:
   i) molecular weight: 19,000 ± 1,000 as measured by electrophoresis through a sodium dodecylsulfate - polyacrylamide gel;
   ii) isoelectric point: having at least one of the three isoelectric points, pl = 5.5 ± 0.1, pl = 5.8 ± 0.1, and pl = 6.1 ± 0.1;
   iii) ultraviolet absorption: having a maximum absorption at 280 nm and a minimum absorption at 250 nm;
   iv) amino acid sequence of the 21 residues from N terminus: 
H2N - Thr - Pro - Leu - Gly - Pro - Ala - Ser - Ser -
Leu - Pro - Gln - Ser - Phe - Leu - Leu - Lys - Cys -
Leu - Glu - Gln - Val -
2) a human G-CSF having a polypeptide represented by all or part of the following amino acid sequence: 
(Met)nThr Pro Leu Gly Pro Ala Ser Ser Leu Pro
Gln Ser Phe Leu Leu Lys Cys Leu Glu Gln Val
Arg Lys Ile Gln Gly Asp Gly Ala Ala Leu Gln
Glu Lys X Cys Ala Thr Tyr Lys Leu Cys His
Pro Glu Glu Leu Val Leu Leu Gly His Ser Leu
Gly Ile Pro Trp Ala Pro Leu Ser Ser Cys Pro
Ser Gln Ala Leu Gln Leu Ala Gly Cys Leu Ser
Gln Leu His Ser Gly Leu Phe Leu Tyr Gln Gly
Leu Leu Gln Ala Leu Glu Gly Ile Ser Pro Glu
Leu Gly Pro Thr Leu Asp Thr Leu Gln Leu Asp
Val Ala Asp Phe Ala Thr Thr Ile Trp Gln Gln
Met Glu Glu Leu Gly Met Ala Pro Ala Leu Gln
Pro Thr Gln Gly Ala Met Pro Ala Phe Ala Ser
Ala Phe Gln Arg Arg Ala Gly Gly Val Leu Val
Ala Ser His Leu Gln Ser Phe Leu Glu Val Ser
Tyr Arg Val Leu Arg His Leu Ala Gln Pro
 (where X is Leu or Leu-Val-Ser-Glu; and n is 0 or l).

Most preferably, either of the two types of human G-CSF takes on the form of glycoprotein having a sugar chain portion.

The G-CSF of type (I) may be prepared by either of the methods described in EP-A-0 169 566 and EP-A-0 217 404. The former application describes a method of isolating the desired human G-CSF from the supernatant of the culture of a cell strain, CHU-I, that was derived from human oral cavity cancer and which has been deposited with Collection Nationale de Cultures de Microorganismes, Institut Pasteur, France under C.N.C.M. Accession Number l-3l5. The latter application describes a method of isolating the desired human G-CSF from the supernatant of the culture of a cell strain, CHU-2, that was also derived from human oral cavity cancer and which has been deposited with C.N.C.M. under Accession Number l-483. For further details of the two methods, see the specifications of the respective applications.

The G-CSF of type (2) may be prepared by either of the methods described in EP-A-0 215 126. All of these methods rely on "DNA recombinant technology". The methods described in the first two applications use E. coli and other procaryotic cells as host cells, and those shown in the other two applications employ animal cells as host cells. For further details of these methods, see the specifications of the respective applications.

The most desirable type of G-CSF which assumes the form of a glycoprotein having a sugar chain portion can be produced by the method using animal cells as hosts. The human G-CSF obtained by either of the methods outlined above may be stored in a frozen state or after being dehydrated by such means as freeze-drying or vacuum drying. If desired, the human G-CSF may be dissolved in an appropriate buffer, followed by aseptic filtration through a Millipore filter or any other suitable means to formulate an injection.

The pharmaceutical agent prepared according to the present invention having the ability to promote the recovery of hemopoietic capacity may contain the pharmaceutical carrier or excipient necessary to assist in its formulation in a dosage form suitable for administration to humans. If desired, a stabilizer and an anti-adsorption agent may also be incorporated in this agent.

The level of dosage and the frequency of administration of the human G-CSF in the pharmaceutical agent of the present invention may be determined in consideration of the severity of the disease to be treated; typically, a dosage containing 0.l - 500 µg, preferably 5 - l00 µg, of human G-CSF may be administered to an adult at a frequency of one to seven times a week. However, it should be noted that the present invention is by no means limited by the content of human G-CSF.

The pharmaceutical agent prepared according to the present invention is efficacious for promoting the recovery of hemopoietic capacity of patients with hemopoietic disorders who have received the therapy of bone marrow transplantation. The present invention will therefore hold much promise for the effective treatment of patients who are suffering from leukemia and other blood diseases that are refractory to conventional therapeutic regimens.

### Examples

The following referential example, experimental examples and working examples are provided for the purpose of illustrating the preparation of G-CSF, its pharmacological effects and its formulation in various dosage forms, respectively, but it should be understood that the scope of the present invention is by no means limited by these examples.

### Referential Example: Preparation of human G-CSF using animal cells (mouse Cl27 cells)

Plasmid, PTN-V2, was obtained by the procedures described in Examples l - l2 of Japanese Patent Application No. 269456/l985, and subsequently treated with BamHI as follows. Twenty micrograms of the plasmid pTN-V2 was dissolved in 200 µl of a reaction solution [10 mM Tris-HCl (pH 8.0), mM MgCl₂, 100 mM NaCl, 2 mM 2-mercaptoethanol and 0.0l% BSA] and treated with 20 units of BamHI (Takara Shuzo Co., Ltd.), followed by treatments with phenol and ether, and precipitation with ethanol.

Mouse Cl27 cells were grown in a Dulbecco's minimal essential medium containing l0% bovine fetal serum (Gibco). The Cl27 cells growing an plates (5 cm diameter) were transformed with l0 µg, per plate, of the separately prepared DNA by the calcium phosphate procedure [see Haynes, J. & Weissmann, C., Nucleic Acids Res., II, 687 - 706 (l983)]. After treatment with glycerol, the cells were incubated at 37°C for l2 hours.

The incubated cells were transferred onto three fresh plates (5 cm diameter) and the media were changed twice a week. At day l6, the foci were transferred onto fresh plates and subjected to serial cultivation on a Dulbecco's minimal essential medium containing l0% bovine fetal serum (Gibco), so as to select clones having a high G-CSF production rate. These clones produced G-CSF at a level of approximately l mg/l.

For the methods of recovering, purifying and assaying the so obtained G-CSF, see the pertinent Examples shown in the specification of Japanese Patent Application No. 269456/l985.

### Experiment I: Correlation between daily administration of G-CSF to mice and their hemopoietic capacity

A portion (0.l ml) of a G-CSF sample (a physiological saline solution containing 2.5 µg of CHU-2 derived G-CSF, l% n-propanol and l0% serum from C57BL mice) was daily administered to 8-week-old male C57BL mice. On predetermined days (see Table I below), the mice were sacrificed and the CFU-C and CFU-S counts in the spleen and the peripheral neutrophile count of each animal were obtained for comparison with the respective values for mice treated with 0.l ml of a control sample (a physiological saline solution containing l% n-propanol and l0% serum from C57BL mice). The results are shown in Tables l, 2 and 3; CFU-S signifies stem cells capable of differentiating to erythrocytes, neutrophiles, megakaryocytes, eosinophiles or monocytes, and CFU-C signifies progenitor cells which are capable of differentiating to neutrophiles and/or monocytes (macrophages) and sometimes to eosinophiles.

As the data in Tables l, 2 and 3 show, the mice which received daily administration of G-CSF exhibited enhanced hemopoietic capacity.

**Table 1**

| CFU-C count in one spleen (number of measurements, n = 4) | | |
|---|---|---|
| Days | Control | G-CSF treated group |
| 0 | 1820.8 ± 592.1 | 1820.8 ± 592.1 |
| 5 | 1619.5 ± 464.7 | 28119 ± 2172.6*** |
| 11 | 1708.5 ± 418.9 | 78318.8 ± 16922.3** |
| P: ***<0.001<**<0.01 | | |

**Table 2**

| CFU-S count in one spleen (n = 4) | | |
|---|---|---|
| Days | Control | G-CSF treated group |
| 0 | 1939 ± 556 | 1939 ± 556 |
| 5 | 2065 ± 47 | 8658 ± 313*** |
| 11 | 1471 ± 409 | 13907 ± 1875** |
| P: ***<0.001<**<0.01 | | |

**Table 3**

| Peripheral neutrophile count in 1 mm³ of peripheral blood (n = 4) | | |
|---|---|---|
| Days | Control | G-CSF treated group |
| 0 | 765 ± 139 | 765 ± 139 |
| 2 | 1344 ± 389 | 3205 ± 439* |
| 5 | 1378 ± 474 | 4913 ± 530** |
| 8 | 1127 ± 242 | 3337 ± 308** |
| 11 | 965 ± 231 | 5229 ± 550*** |
| P: ***<0.001<**<0.01<*<0.05 | | |

### Experiment 2: Ability of G-CSF to promote the recovery of hemopoietic capacity after BMT

Mice (C57BL, 8-week-old, male) were subjected to total-body irradiation with 950 rad of X-rays. Immediately thereafter, 2 x 10⁵ bone marrow cells of C57BL mice were transplanted by injection through the tail vein. Starting on 5 days after the transplantation, 0.1 ml of the control or G-CSF sample used in Experiment 1 was administered daily, and on 6 and 12 days after the administration, the CFU-S counts in the spleen and bone marrow were performed. The results are shown in Tables 4 and 5.

**Table 4**

| CFU-S count in the spleen (n = 4) | | |
|---|---|---|
| | Control | G-CSF treated group |
| normal mice | 1664 ± 371 | |
| day 6 | 34 ± 34 | 843 ± 504 |
| day 12 | 230 ± 230 | 6116 ± 3531 |

**Table 5**

| CFU-S count in bone marrow (for one thigh bone) (n = 4) | | |
|---|---|---|
| | Control | G-CSF treated group |
| normal mice | 2852 ± 344 | |
| day 6 | 33 ± 22 | 51 ± 24 |
| day 12 | 128 ± 57 | 254 ± 165 |

### Experiment 3: Survival rate in animal model with delayed recovery of hemopoietic ability

Mice (C57BL, 8-week-old, male) were subjected to total-body irradiation with 950 rad of X-rays. Immediately thereafter, 7.5 x 10⁴ bone marrow cells of C57BL mice were transplanted by injection through the tail vein. Starting on 5 days after the transplantation, 0.1 ml of the control or G-CSF sample used in Experiment 1 was administered daily for 11 consecutive days. The survival rates of the two groups of mice on 40 days after the X-ray irradiation were as follows.

Control group 33.3% (n = l2)
G-CSF treated group 75.0% (n = l2)
The significant improvement in survival rate is believed to be attributable to the ability of G-CSF to promote the recovery of hemopoietic capacity.

### Example l

The human G-CSF prepared in the Referential Example was rendered germ-free and frozen at -20°C. The frozen fraction was worked up to prepare an injection.

### Example 2

The human G-CSF prepared in the Referential Example was aseptically charged in 5 ml portions in l0 ml vials and freeze-dried at -20°C, with the vials being subsequently closed with rubber stopper. The so obtained freeze-dried products were worked up to prepare an injection.

## Claims

1. The use of a human granulocyte colony stimulating factor in an amount from 0.1 to 500 µg for the production of a pharmaceutical composition for the recovery of hematopoietic capacity following bone marrow transplantation by promoting the increase of CFU-S.

2. The use according to Claim 1, wherein the amount of human granulocyte colony stimulating factor is from 5 to 100 µg.

3. The use according to Claim 1 or 2, wherein said human granulocyte colony stimulating factor has the following physicochemical properties:
i) molecular weight: 19,000 ± 1,000 as measured by electrophoresis through a sodium dodecylsulfate-polyacrylamide gel;
ii) isoelectric point: having at least one of the three isoelectric points, pI = 5.5 ± 0.1, pI = 5.8 ± 0.1, and pI = 6.1 ± 0.1;
iii) ultraviolet absorption: having a maximum absorption at 280 nm and a minimum absorption at 250 nm;
iv) amino acid sequence of the 21 residues from N terminus:

H2N - Thr - Pro - Leu - Gly - Pro - Ala - Ser - Ser -
Leu - Pro - Gln - Ser - Phe - Leu - Leu - Lys - Cys -
Leu - Glu - Gln - Val -.

4. The use according to any one of claims 1 to 3, wherein the polypeptide having the human granulocyte colony stimulating factor activity is represented by all or part of the amino acid sequence shown below:

(Met)nThr Pro Leu Gly Pro Ala Ser Ser Leu Pro
Gln Ser Phe Leu Leu Lys Cys Leu Glu Gln Val
Arg Lys Ile Gln Gly Asp Gly Ala Ala Leu Gln
Glu Lys X Cys Ala Thr Tyr Lys Leu Cys His
Pro Glu Glu Leu Val Leu Leu Gly His Ser Leu
Gly Ile Pro Trp Ala Pro Leu Ser Ser Cys Pro
Ser Gln Ala Leu Gln Leu Ala Gly Cys Leu Ser
Gln Leu His Ser Gly Leu Phe Leu Tyr Gln Gly
Leu Leu Gln Ala Leu Glu Gly Ile Ser Pro Glu
Leu Gly Pro Thr Leu Asp Thr Leu Gln Leu Asp
Val Ala Asp Phe Ala Thr Thr Ile Trp Gln Gln
Met Glu Glu Leu Gly Met Ala Pro Ala Leu Gln
Pro Thr Gln Gly Ala Met Pro Ala Phe Ala Ser
Ala Phe Gln Arg Arg Ala Gly Gly Val Leu Val
Ala Ser His Leu Gln Ser Phe Leu Glu Val Ser
Tyr Arg Val Leu Arg His Leu Ala Gln Pro

(wherein X is Leu or Leu-Val-Ser-Glu; and n is 0 or 1).

## Patentansprüche

1. Verwendung eines menschlichen Granulocyten-Kolonie-stimulisierenden Faktors in einer Menge von 0,1 - 500 µg zur Herstellung eines Arzneimittels zur Wiederherstellung der hämatopoietischen Fähigkeit nach einer Knochenmarkstransplantation durch Förderung des Anstiegs von CFU-S.

2. Verwendung gemäß Anspruch 1, wobei die Menge des Granulocyten-Kolonie-stimulisierenden Faktors des Menschen von 5 bis 100 µg beträgt.

3. Verwendung gemäß Anspruch 1 oder 2, wobei der Granulocyten-Kolonie-stimulisierende Faktor des Menschen die folgenden physikalisch chemischen Eigenschaften aufweist:
i) Molekulargewicht: 19 000 ± 1000, gemessen durch Elektrophorese durch ein Natrium-dodecylsulfat-Polyacrylamid-Gel;
ii) isoelektrischer Punkt: Er weist mindestens einen der drei isoelektrischen Punkte auf, pI = 5,5 ± 0,1, pI = 5,8 ± 0,1, und pI = 6,1 ± 0,1;
iii) UV-Absorption: Er hat ein Absorptionsmaximum bei 280 nm und ein Absorptionsminimum bei 250 nm,
iv) Aminosäuresequenz der 21 Reste vom N-Terminus:

H2N - Thr - Pro - Leu - Gly - Pro - Ala - Ser - Ser -
Leu - Pro - Gln - Ser - Phe - Leu - Leu - Lys - Cys -
Leu - Glu - Gln - Val -

4. Verwendung gemäß einem der Ansprüche 1 bis 3, wobei das den Granulocyten-Kolonie-stimulisierenden Faktor des Menschen enthaltende Polypeptid durch die gesamte oder einen Teil der nachstehend aufgeführten Aminosäuresequenz wiedergegeben wird:

(Met)nThr Pro Leu Gly Pro Ala Ser Ser Leu Pro
Gln Ser Phe Leu Leu Lys Cys Leu Glu Gln Val
Arg Lys Ile Gln Gly Asp Gly Ala Ala Leu Gln
Glu Lys X Cys Ala Thr Tyr Lys Leu Cys His
Pro Glu Glu Leu Val Leu Leu Gly His Ser Leu
Gly Ile Pro Trp Ala Pro Leu Ser Ser Cys Pro
Ser Gln Ala Leu Gln Leu Ala Gly Cys Leu Ser
Gln Leu His Ser Gly Leu Phe Leu Tyr Gln Gly
Leu Leu Gln Ala Leu Glu Gly Ile Ser Pro Glu
Leu Gly Pro Thr Leu Asp Thr Leu Gln Leu Asp
Val Ala Asp Phe Ala Thr Thr Ile Trp Gln Gln
Met Glu Glu Leu Gly Met Ala Pro Ala Leu Gln
Pro Thr Gln Gly Ala Met Pro Ala Phe Ala Ser
Ala Phe Gln Arg Arg Ala Gly Gly Val Leu Val
Ala Ser His Leu Gln Ser Phe Leu Glu Val Ser
Tyr Arg Val Leu Arg His Leu Ala Gln Pro

(wobei X Leu oder Leu-Val-Ser-Glu ist und n 0 oder 1
ist.

## Revendications

1. Utilisation d'un facteur de stimulation de colonie des granulocytes humain en une quantité de 0,1 à 500 µg, pour la fabrication d'une composition pharmaceutique pour la récupération de la capacité hematopoïétique après une transplantation de moelle osseuse, en favorisant l'augmentation des CFU-S.

2. Utilisation suivant la revendication 1, dans laquelle la quantité du facteur de stimulation de colonie des granulocytes humain est de 5 à 100 µg.

3. Utilisation suivant la revendication 1 ou 2, dans laquelle le facteur de stimulation de colonie des granulocytes humain a les propriétés physico-chimiques suivantes :
(i) poids moléculaire : 19 000 ± 1000 tel que mesuré par électrophorèse sur du gel de polyacrylamide avec dodécylsulfate de sodium;
(ii) point isoélectrique : ayant au moins l'un des trois points isoélectriques, pI = 5,5 ± 0,1, pI = 5,8 ± 0,1 et pI = 6,1 ± 0,1;
(iii) absorption dans l'ultraviolet : ayant un maximum d'absorption à 280 nm et un minimum d'absorption à 250 nm;
(iv) séquence des acides aminés des 21 résidus à partir de l'extrémité N terminale :

H2N - Thr - Pro - Leu - Gly - Pro - Ala - Ser - Ser -
Leu - Pro - Gln - Ser - Phe - Leu - Leu - Lys - Cys -
Leu - Glu - Gln - Val -

4. Utilisation suivant l'une quelconque des revendications 1 à 3, dans laquelle le polypeptide ayant le facteur de stimulation de colonie des granulocytes humain est représenté par tout ou partie de la séquence ci-après d'acides aminés :
